(19) Europäisches Patentamt
European Patent Office
Office européen des brevets

(11) Veröffentlichungsnummer: **0 504 695 A1**

# (12) EUROPÄISCHE PATENTANMELDUNG

(21) Anmeldenummer: **92103970.7**

(22) Anmeldetag: **09.03.92**

(51) Int. Cl.[5]: **C07D 209/34**, C07D 215/38, C07D 215/48, C07D 223/16, C07D 241/44, C07D 243/12, C07D 245/06, A61K 31/40, A61K 31/47, A61K 31/495, A61K 31/55

(30) Priorität: **21.03.91 CH 863/91**

(43) Veröffentlichungstag der Anmeldung: **23.09.92 Patentblatt 92/39**

(84) Benannte Vertragsstaaten: **AT BE CH DE DK ES FR GB GR IT LI LU MC NL PT SE**

(71) Anmelder: **F. HOFFMANN-LA ROCHE AG**
**Grenzacherstrasse 124**
**CH-4002 Basel(CH)**

(72) Erfinder: **Klaus, Michael**
**Am Hellenrain 6**
**W-7858 Weil/Rhein(DE)**
Erfinder: **Mohr, Peter**
**Martinsgasse 9**
**CH-4051 Basel(CH)**

(74) Vertreter: **Grossner, Lutz, Dr. et al**
**Grenzacher Strasse 124 Postfach 3255**
**CH-4002 Basel(CH)**

## (54) Neue Carbonsäureamide, ihre Herstellung und pharmazeutische Präparate, die diese Verbindungen enthalten.

(57) Die neuen Carbonsäureamide und Anilide der Formel

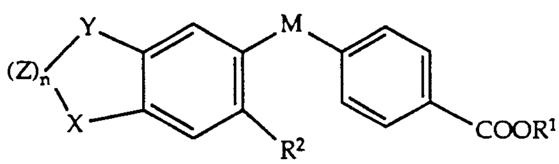

I

worin

| | |
|---|---|
| $R^1$ | Wasserstoff, nieder-Alkyl oder ein Kation; |
| $R^2$ | Wasserstoff, nieder-Alkyl, nieder-Alkoxy oder Halogen; |
| M | -CONH- oder -NHCO-; |
| X und Y | $>CR^3R^4$ oder $-CONR^5-$; |
| Z | $>CR^6R^7$; |
| $R^3$, $R^4$, $R^6$ und $R^7$ | Wasserstoff oder nieder-Alkyl; |
| $R^5$ | Alkyl; und |
| n | 0,1 oder 2 bedeuten; |

wobei mindestens ein Rest X oder Y $-CONR^5-$ ist und dieser Rest über das N-Atom an den Phenylring gebunden ist,
können bei der Behandlung und Prophylaxe von Neoplasien, Dermatosen und Altershaut Verwendung finden.

EP 0 504 695 A1

Die vorliegende Erfindung betrifft neue Carbonsäureamide und Anilide der Formel

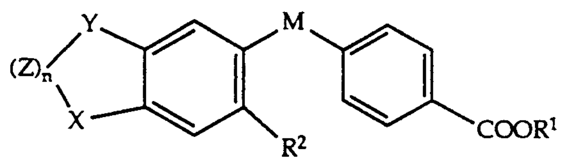

I

worin

| | |
|---|---|
| $R^1$ | Wasserstoff, nieder-Alkyl oder ein Kation; |
| $R^2$ | Wasserstoff, nieder-Alkyl, nieder-Alkoxy oder Halogen; |
| M | -CONH- oder -NHCO-; |
| X und Y | $>CR^3R^4$ oder $-CONR^5-$; |
| Z | $>CR^6R^7$; |
| $R^3$, $R^4$, $R^6$ und $R^7$ | Wasserstoff oder nieder-Alkyl; |
| $R^5$ | Alkyl; und |
| n | 0, 1 oder 2 bedeuten; |

wobei mindestens ein Rest X oder Y $-CONR^5-$ ist und dieser Rest über das N-Atom an den Phenylring gebunden ist.

Die Erfindung betrifft weiterhin ein Verfahren zur Herstellung der Verbindungen der Formel I, pharmazeutische Präparate auf Basis der Verbindungen der Formel I, die Verbindungen der Formel I bei der Behandlung und Prophylaxe von Neoplasien, Dermatosen und Altershaut sowie die Verwendung der Verbindungen der Formel I bei der Herstellung von pharmazeutischen Präparaten zur Behandlung und Prophylaxe solcher Erkrankungen. Grn/7.1.92

Die Bezeichnung "nieder" bezieht sich auf Gruppen mit 1-6 C-Atomen. Alkyl- und Alkoxygruppen können geradkettig oder verzweigt sein, wie Methyl, Aethyl, Propyl, Isopropyl, n-Butyl, sek.-Butyl oder tert.-Butyl bzw. Methoxy, Aethoxy, Propoxy, Isopropoxy, n-Butoxy, sek- oder tert.-Butoxy. Halogen umfasst Fluor, Chlor, Brom und Jod.

Die Carbonsäuren der Formel I bilden mit Basen, insbesondere mit den Alkalimetallhydroxiden, vorzugsweise mit Natrium- oder Kaliumhydroxid, Salze, die ebenfalls Gegenstand der Erfindung sind.

Eine bevorzugte Gruppe von Verbindungen der Formel I sind die, in denen n 1 oder 2, insbesondere 2, ist. Weiterhin bevorzugt sind Verbindungen der Formel I, worin einer der Reste X und Y-$CONR^5$ und der andere $>CR^3R^4$ darstellt. $R^1$ und $R^2$ sind vorzugsweise Wasserstoff; $R^3$ und $R^4$ sind vorzugsweise nieder-Alkyl; $R^5$ ist vorzugsweise Methyl; $R^6$ und $R^7$ sind vorzugsweise Wasserstoff.

Die Verbindungen der Formel I können dadurch hergestellt werden, dass man eine Verbindung der Formel

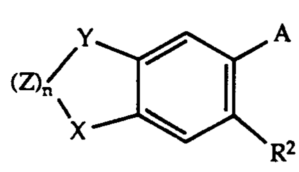

II

mit einer Verbindung der Formel

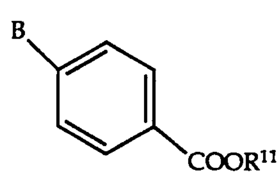

III

umsetzt;

oder eine Verbindung der Formel

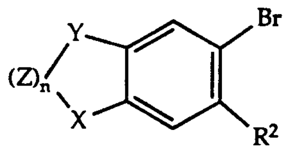

IV

mit einer Verbindung der Formel

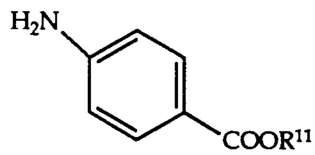

V

und Kohlenmonoxid in Gegenwart eines Uebergangsmetalls und eines Amins umsetzt,

wobei entweder A eine Carboxylgruppe oder ein reaktionsfähiges Derivat davon und B eine Aminogruppe bedeutet, oder A eine Aminogruppe und B eine Carboxylgruppe oder ein reaktionsfähiges Derivat davon und $R^{11}$ nieder-Alkyl bedeutet,

und gewünschtenfalls die Estergruppe $COOR^{11}$ verseift und die gebildete Säure als solche oder als Salz isoliert.

Die Umsetzung einer Verbindung II mit einer Verbindung III kann nach an sich bekannten Methoden für die Acylierung von Aminen durchgeführt werden. Vorzugsweise wird eine Verbindung der Formel II, in der A eine Carbonsäurehalogenidgruppe, z.B. die Gruppe -COCl darstellt, mit einer Verbindung der Formel III, in der B $-NH_2$ ist, zu einer Verbindung der Formel I, in der M -CONH- ist, umgesetzt, oder ein Amin der Formel II mit einem Carbonsäurehalogenid der Formel III zu einer Verbindung der Formel I umgesetzt, in der M -NHCO- ist.

Diese Acylierungen werden zweckmässig in Gegenwart einer Base, z.B. einer organischen Base, wie Pyridin durchgeführt.

Als Uebergangsmetall-Katalysatoren für die Umsetzung von Verbindungen der Formel IV und V mit Kohlenmonoxid kommen z.B. Nickel und insbesondere Palladium in Betracht. Als Amine kommen insbesondere tertiäre Amine, wie Tributylamin und Triäthylamin in Betracht, die gleichzeitig als Lösungsmittel für die Reaktionspartner dienen können. Gewünschtenfalls kann ein zusätzliches, inertes organisches Lösungsmittel, wie Dimethylformamid oder Dimethylacetamid eingesetzt werden. Die Reaktion wird zweckmässig bei erhöhter Temperatur, z.B. bei 50-100 ° C und unter erhöhtem CO-Druck, z.B. einem CO-Druck von 5-20 bar, durchgeführt.

Ein Carbonsäureester der Formel I kann in an sich bekannter Weise, z.B. durch Behandlung mit Alkalien, insbesondere durch Behandeln mit wässriger alkoholischer Natron- oder Kalilauge, in einem zwischen Raumtemperatur und dem Siedepunkt des Reaktionsgemisches liegenden Temperaturbereich zur Carbonsäure hydrolysiert werden. Die Carbonsäure der Formel I kann in freier Form isoliert werden oder in ein pharmazeutisch anwendbares Salz, z.B. das Na- oder K-Salz, übergeführt werden.

Die als Ausgangsmaterial für die Herstellung der Verbindungen der Formel I verwendeten Verbindungen der Formeln II und III können, soweit sie nicht bekannt oder nachstehend beschrieben sind, in Analogie zu bekannten oder den nachstehend beschriebenen Methoden hergestellt werden.

Die Verfahrensprodukte der Formel I und ihre physiologisch verträglichen Salze stellen pharmakodynamisch wertvolle Verbindungen dar. Sie können zur topischen und systemischen Therapie von benignen und malignen Neoplasien, von prämalignen Läsionen, sowie ferner auch zur systemischen und topischen Prophylaxe der genannten Affektionen verwendet werden.

Sie sind des weiteren für die topische und systemische Therapie von Akne, Psoriasis und anderen mit einer verstärkten oder pathologisch veränderten Verhornung einhergehenden Dermatosen, wie auch von entzündlichen und allergischen dermatologischen Affektionen sowie lichtgeschädigter (Alters)haut geeignet. Die Verfahrensprodukte der Formel I können ferner auch zur Bekämpfung von Schleimhauterkrankungen mit entzündlichen oder degenerativen bzw. metaplastischen Veränderungen eingesetzt werden.

Die Verbindungen der Formel I können auch zur Behandlung entzündlicher, allergischer, rheumatischer und immunologischer Erkrankungen der verschiedensten Organe verwendet werden. Beispiele solcher Krankheiten sind: Primär-chronische Polyarthritis, Spondylarthritis ancylopoetica, Osteoarthritiden, Arthritiden und Arthrosen; Ekzeme, atopische Dermatitis, Rhinitis allergica, Asthma bronchiale; Autoimmunkrankheiten wie z.B. Lupus erythematosus, Reiter's Syndrom. Im Gegensatz zu Retinoiden konnte bei den vorliegenden Verbindungen in Zellkulturen von embryonalen Beinanlagen (Arch. Toxicol. (1987) 60:403) keine teratogene Wirksamkeit festgestellt werden.

Die Wirksamkeit der Verbindungen der Formel I bei der Behandlung von Akne kann z.B. in-vitro anhand der anti-proliferativen Wirkung auf menschliche Sebocyten (J. Invest. Dermatol. 90(1988)554) oder durch Beeinflussung der Talgdrüsen am Ohr des männlichen syrischen Goldhamsters (J. Invest. Dermatol. 81 (1983) 43 und 68 (1977) 171) geprüft werden. Bei der Hemmung der Sebocyten-Proliferation zeigten die geprüften Verbindungen der Formel I Hemmwirkung ($ED_{50}$) in einer Konzentration von 0,1-1 $\mu$Mol.

Bei der Prüfung im Hamsterohr-Modell wurden mit den geprüften Verbindungen der Formel I mit Dosierungen von 0,5 bis 10 mg/kg Verminderungen des mittleren Drüsenquerschnitts zwischen etwa 20 und 40% beobachtet.

Die Verbindungen der Formel I und deren Salze können deshalb als Heilmittel, z.B. in Form pharmazeutischer Präparate, Anwendung finden.

Die Mittel können enteral, parenteral oder topisch verabreicht werden. Für die enterale Applikation eignen sich z.B. Mittel in Form von Tabletten, Kapseln, Dragées, Sirupen, Suspensionen, Lösungen und Suppositorien. Für die parenterale Applikation sind Mittel in Form von Infusions- oder Injektionslösungen geeignet.

Die Dosierungen, in denen die Präparate verabreicht werden, können je nach Anwendungsart und Anwendungsweg sowie nach den Bedürfnissen der Patienten variieren.

Bei oraler Verabreichung der erfindungsgemässen Verbindungen kommen beim Erwachsenen Dosierungen von etwa 0,1-100 mg/kg, vorzugsweise 0,5-50 mg/kg pro Tag, in Betracht.

Die Präparate können in einer oder mehreren Dosierungen verabreicht werden. Eine bevorzugte Darreichungsform sind Kapseln mit einem Gehalt von ca. 5-500 mg Wirkstoff.

Die Präparate können inerte oder auch pharmakodynamisch aktive Zusätze enthalten. Tabletten oder Granulate z.B. können eine Reihe von Bindemitteln, Füllstoffen, Trägersubstanzen oder Verdünnungsmitteln enthalten. Flüssige Präparate können beispielsweise in Form einer sterilen, mit Wasser mischbaren Lösung vorliegen. Kapseln können neben dem Wirkstoff zusätzlich ein Füllmaterial oder Verdickungsmittel enthalten. Des weiteren können geschmacksverbessernde Zusätze sowie die üblicherweise als Konservierungs-, Stabilisierungs, Feuchthalte- und Emulgiermittel verwendeten Stoffe, ferner auch Salze zur Veränderung des osmotischen Druckes, Puffer und andere Zusätze vorhanden sein.

Die vorstehend erwähnten Trägersubstanzen und Verdünnungsmittel können aus organischen oder anorganischen Stoffen, z.B. aus Wasser, Gelatine, Milchzucker, Stärke, Magnesiumstearat, Talkum, Gummi arabicum, Polyalkylenglykolen und dergleichen bestehen. Voraussetzung ist, dass alle bei der Herstellung der Präparate verwendeten Hilfsstoffe untoxisch sind.

Zur topischen Anwendung werden die Wirkstoffe zweckmässig in Form von Salben, Tinkturen, Crèmen, Lösungen, Lotionen, Sprays, Suspensionen und dergleichen verwendet. Bevorzugt sind Salben und Crèmen sowie Lösungen. Diese zur topischen Anwendung bestimmten Präparate können dadurch hergestellt werden, dass man die Verfahrensprodukte als wirksamen Bestandteil nichttoxischen, inerten, für topische Behandlung geeigneten, an sich in solchen Präparaten üblichen festen oder flüssigen Trägern zumischt.

Für die topische Anwendung sind zweckmässig ca. 0,1-5%ige, vorzugsweise 0,3-2%ige Lösungen, sowie ca. 0,1-5%ige, vorzugsweise ca. 0,3-2%ige Salben oder Crèmen geeignet.

Den Präparaten kann gegebenenfalls ein Antioxydationsmittel, z.B. Tocopherol, N-Methyl-$\gamma$-tocopheramin sowie t-Butyl-hydroxyanisol oder t-Butyl-hydroxytoluol beigemischt sein.

## Beispiel 1

A. Eine Lösung von 25,0 g 4,4-Dimethyltetralon in 180 ml Eisessig wurde mit 15,8 g Natriumazid versetzt, auf 80°C erwärmt und die Lösung innerhalb von 1 Stunde mit 25 ml konz. Schwefelsäure versetzt. Danach wurde das Reaktionsgemisch noch 30 Minuten gerührt und dann auf ein Gemisch von Eis und konz. Natronlauge gegossen. Extraktion mit Natriumacetat, Waschen mit Natriumchloridlösung, Trocknen und Eindampfen lieferte 32 g eines Rohprodukts, das unmittelbar weiter umgesetzt wurde.

8,85 g Natriumhydrid (ca. 55%ig) wurden unter Argon in 100 ml Dimethylformamid suspendiert. Nach Kühlen auf 0°C wurde eine Lösung von 32 g des vorstehend erhaltenen Rohprodukts in 100 ml Dimethylformamid zugesetzt und es wurde 1 Stunde gerührt. Danach wurden 15,8 ml Methyljodid zugegeben und das Kühlbad wurde entfernt. Nach Abklingen der stark exothermen Reaktion wurde auf Eis gegossen, mit Diäthyläther extrahiert, mit Natriumchlorid gewaschen, getrocknet und eingedampft. Flash-Chromatographie an Kieselgel (Petroläther/Aethylacetat 7:3) lieferte 16,0 g reines 2,3,4,5-Tetrahydro-1,5,5-trimethyl-2-oxo-1H-1-benzazepin als farbloses Oel.

B. Zu 7,1 ml Acetanhydrid wurden bei 0 °C vorsichtig 2,0 ml 65%ige Salpetersäure getropft. Diese Lösung wurde zu einer Lösung von 3,14 g 2,3,4,5-Tetrahydro-1,5,5-trimethyl-2-oxo-1H-1-benzazepin in 9,0 ml Acetanhydrid und 2,8 ml Eisessig gegeben. Das Reaktionsgemisch wurde 3 Tage bei Raumtemperatur stehengelassen und dann auf Eis gegossen, nacheinander mit Wasser, Sodalösung und Wasser gewaschen, getrocknet und eingedampft. Umkristallisation aus Aethylacetat/Hexan lieferte 3,39 g 2,3,4,5-Tetrahydro-1,5,5-trimethyl-7-nitro-2-oxo-1H-1-benzazepin, Smp. 113-115 °C.

C. 3,33 g der vorstehend erhaltenen Nitroverbindung wurden in 190 ml abs. Aethanol gelöst und bei Normaldruck über 230 mg Palladium/Kohle (5%) bei Raumtemperatur hydriert. Nach 1,5 Stunden wurde der Katalysator abfiltriert und das Lösungsmittel unter vermindertem Druck entfernt. Man erhielt 2,92 g 7-Amino-2,3,4,5-tetrahydro-1,5,5-trimethyl-2-oxo-1H-1-benzazepin, Smp. 119-122 °C.

D. Aus 2,86 g Terephthalsäuremonoäthylester wurde durch Rückflusserhitzen mit 1,94 ml $SOCl_2$ das entsprechende Säurechlorid hergestellt, das nach sorgfältigem Trocknen in 25 ml Pyridin gelöst und unter Argon bei 0 °C tropfenweise mit einer Lösung von 2,91 g des in Abschnitt C. erhaltenen Amins in 12 ml Pyridin versetzt wurde. Nach 30 Minuten bei Raumtemperatur wurde das Reaktionsgemisch auf Eis/konz. Salzsäure gegossen und mit Aethylacetat extrahiert. Nach Waschen mit Wasser, Sodalösung und Wasser, Trocknen und Eindampfen wurden 4,24 g Aethyl-p-[(2,3,4,5-tetrahydro-1,5,5-trimethyl-2-oxo-1H-1-benzazepin-7-yl)carbamoyl]benzoat als farblose Kristalle, Smp. 224-226 °C (aus Aethylacetat) erhalten.

Beispiel 2

A. Zu einer bei 0 °C aus 26,4 ml Diisopropylamin und 116 ml 1,6M n-Butyllithium in Hexan hergestellten Lithium-diisopropylamin-Lösung in 220 ml abs. Tetrahydrofuran wurden nach Kühlen auf -78 °C eine Lösung von 27,8 g 2,3,4,5-Tetrahydro-1-methyl-2-oxo-1H-1-benzazepin in 50 ml abs. Tetrahydrofuran getropft. Das Reaktionsgemisch wurde auf Raumtemperatur aufgewärmt, danach erneut auf -78 °C gekühlt und mit 19,4 ml Methyljodid versetzt. Danach wurde das Kühlbad entfernt und das Reaktionsgemisch noch 30 Minuten gerührt, das Reaktionsgemisch auf Eis/konz. Salzsäure gegossen, mit Diäthyläther extrahiert, mit Wasser und gesättigter Kochsalzlösung gewaschen, getrocknet und eingedampft. Man erhielt 28,7 g eines Rohprodukts, das in der vorstehend beschriebenen Weise erneut alkyliert wurde. Das so erhaltene Rohprodukt wurde durch Flash-Chromatographie an Kieselgel (Petroläther/Aethylacetat 85:15) gereinigt und lieferte 18,1 g reines 2,3,4,5-Tetrahydro-1,3,3-trimethy-2-oxo-1H-1-benzazepin als farbloses Oel.

B. Eine Lösung von 18,0 g 2,3,4,5-Tetrahydro-1,3,3-trimethyl-2-oxo-1H-1-benzazepin in 250 ml Methylenchlorid wurde mit 2,0 g Eisenpulver und danach tropfenweise mit 5,83 ml Brom in 30 ml Methylenchlorid versetzt. Das Reaktionsgemisch wurde über Nacht zum Rückfluss erhitzt, danach auf Eis gegossen, mit Diäthyläther extrahiert, mit Natriumpyrosulfitlösung, Wasser und gesättigter Natriumchloridlösung gewaschen, getrocknet und eingedampft. Kristallisation aus Aethylacetat/n-Hexan lieferte 21,4 g reines 7-Brom-2,3,4,5-tetrahydro-1,3,3-trimethyl-2-oxo-1H-1-benzazepin.

C. Eine Lösung von 3,7 g der in Absatz B. erhaltenen Bromverbindung in 80 ml abs. Tetrahydrofuran wurde unter Argon bei -78 °C tropfenweise mit 9,5 ml 1,6M n-Butyllithium in Hexan versetzt. Nach 15 Minuten Reaktionsdauer wurde während 10 Minuten ein kräftiger $CO_2$-Strom durch das Reaktionsgemisch geleitet, danach wurde das Reaktionsgemisch auf Eis/konz. Salzsäure gegossen und mit Aethylacetat extrahiert. Zur weiteren Reinigung wurde der Extrakt mit 1N Natronlauge extrahiert, die wässrige Phase mit konz. Salzsäure auf pH <1 gestellt und erneut mit Aethylacetat extrahiert. Der organische Extrakt wurde mit Wasser und gesättigter Natriumchloridlösung gewaschen, getrocknet und eingedampft. Man erhielt 2,50 g 2,3,4,5-Tetrahydro-1,3,3-trimethyl-2-oxo-1H-1-benzazepin-7-yl-carbonsäure, Smp. 240-241 °C (aus Aethylacetat).

D. 2,38 g 2,3,4,5-Tetrahydro-1,3,3-trimethyl-2-oxo-1H-1-benzazepin-7-yl-carbonsäure wurden durch 30 Minuten Rückflusserhitzen mit 1,75 ml Thionylchlorid in das entsprechende Säurechlorid überführt, nach sorgfältigem Trocknen in 20 ml abs. Pyridin gelöst und bei 0 °C tropfenweise zu einer Lösung von 1,50 g 4-Aminobenzoesäureäthylester in 20 ml abs. Pyridin gegeben. Nach 1 Stunde Reaktionszeit bei Raumtemperatur wurde das Reaktionsgemisch auf Eis/konz. Salzsäure gegossen, mit Aethylacetat extrahiert und die organische Phase nacheinander mit Wasser, 0,5N Natronlauge und gesättigter Kochsalzlösung gewaschen, getrocknet und eingedampft. Man erhielt 2,95 g reines Aethyl-p-(2,3,4,5-tetrahydro-1,3,3-trimethyl-2-oxo-1H-1-benzazepin-7-carboxamido)benzoat, Smp. 169-170 °C (aus Aethylacetat).

Beispiel 3

A. Zu einer unter Argon frisch hergestellten Natriumäthylatlösung aus 10,5 g Natrium und 400 ml abs. Aethanol wurden 43,0 g Dimethylmalonsäurediäthylester und danach 24,7 g o-Phenylendiamin gegeben. Das Reaktionsgemisch wurde im Verlauf von 2 Stunden kontinuierlich auf 180°C erwärmt, wobei Aethanol laufend abdestilliert wurde. Der trockene Rückstand wurde nach dem Erkalten mit verdünnter Salzsäure verrieben, der Niederschlag abfiltriert und mit Wasser und Aethanol gewaschen. Nach Trocknen im Hochvakuum erhielt man 30,2 g 2,3,4,5-Tetrahydro-3,3-dimethyl-2,4-dioxo-1H-1,5-benzodiazepin als bräunliches Pulver, Smp. >270°C. Dieses Produkt wurde portionsweise unter Argon zu 17,0 g Natriumhydrid (ca. 55%ig) in 300 ml abs. Dimethylformamid bei 0°C gegeben. Nach Abklingen der exothermen Reaktion wurden 27,6 ml Methyljodid zugegeben und das Reaktionsgemisch auf Raumtemperatur erwärmt. Danach wurde auf Eis gegossen und mit Aethylacetat extrahiert. Der Extrakt wurde mit Wasser und gesättigter Natriumchloridlösung gewaschen, getrocknet und bis zur Kristallisation eingeengt. Man erhielt 29,5 g 2,3,4,5-Tetrahydro-1,3,3,5-tetramethyl-2,4-dioxo-1H-1,5-benzodiazepin, Smp. 159-160°C.

B. Zu 15 g des vorstehend erhaltenen Benzodiazepin-dions in 200 ml Methylenchlorid wurden unter Argon 1,0 g Eisenpulver und tropfenweise 6,65 ml Brom gegeben. Das Reaktionsgemisch wurde über Nacht bei Raumtemperatur gerührt, danach nochmals mit 3,33 ml Brom und 0,50 g Eisenpulver versetzt und weitere 3 Stunden reagieren gelassen. Danach wurde auf Eis gegossen, mit Aethylacetat extrahiert, mit Pyrosulfitlösung, Wasser und gesättigter Kochsalzlösung gewaschen, getrocknet und bis zur Kristallisation eingeengt. Man erhielt 10,9 g 7-Brom-2,3,4,5-tetrahydro-1,3,3,5-tetramethyl-2,4-dioxo-1H-1,5-benzodiazepin, Smp. 194,5-195°C.

C. Eine Lösung von 5,00 g des vorstehend erhaltenen Bromids in 200 ml abs. Tetrahydrofuran wurde unter Argon bei -78°C tropfenweise mit 12,1 ml 1,6M n-Butyllithium in Hexan versetzt. Nach 30 Minuten wurde das Kühlbad entfernt und 10 Minuten lang ein kräftiger $CO_2$-Strom eingeleitet. Danach wurde das Reaktionsgemisch auf Eis/konz. Salzsäure gegossen, mit Aethylacetat extrahiert, der Extrakt mit Wasser gewaschen, getrocknet und eingedampft. Man erhielt 2,71 g 2,3,4,5-Tetrahydro-1,3,3,5-tetramethyl-2,4,-dioxo-1H-1,5-benzodiazepin-7-yl-carbonsäure, Smp. >275°C.

D. Aus 3,17 g der in Absatz C. erhaltenen Carbonsäure wurde durch 30 Minuten Rückflusserhitzen mit 2,6 ml Thionylchlorid das Säurechlorid hergestellt. Nach sorgfältigem Trocknen wurde das Säurechlorid in 30 ml abs. Pyridin gelöst und unter Argon bei 0°C zu einer Lösung von 1,98 g 4-Aminobenzesäureäthylester in 15 ml abs. Pyridin getropft. Das Reaktionsgemisch wurde 30 Minuten bei Raumtemperatur reagieren gelassen, danach auf Eis/konz. Salzsäure gegossen, mehrmals mit viel Aethylacetat extrahiert, der Extrakt mit Wasser gewaschen, getrocknet und eingedampft. Man erhielt 3,98 g Aethyl-p-(2,3,4,5-tetrahydro-1,3,3,5-tetramethyl-2,4-dioxo-1H-1,5-benzodiazepin-7-carboxamido)benzoat, Smp. 278-279°C.

## Beispiel 4

A. Zu 5,1 ml Acetanhydrid wurden bei 0°C vorsichtig 1,3 ml Salpetersäure (65%ig) getropft. Die Lösung wurde zu einer Lösung von 3,00 g 2,3,4,5-Tetrahydro-1,3,3,5-tetramethyl-2,4-dioxo-1H-1,5-benzodiazepin in 6,5 ml Acetanhydrid und 2,1 ml Eisessig gegeben. Das Reaktionsgemisch wurde 3 Tage bei Raumtemperatur stehengelassen, abfiltriert, der Rückstand gründlich mit Wasser gewaschen, getrocknet und aus Aethanol umkristallisiert. Man erhielt 2,11 g 2,3,4,5-Tetrahydro-1,3,3,5-tetramethyl-7-nitro-2,4-dioxo-1H-1,5-benzodiazepin, Smp. 224-225°C.

B. 2,35 g der in Absatz A. erhaltenen Nitroverbindung wurden in 100 ml abs. Aethanol suspendiert und nach Zusatz von 0,5 g Palladiumkohle (5%) über Nacht bei Raumtemperatur und Normaldruck hydriert. Nach Abfiltrieren über ein Filterhilfsmittel, Waschen und Eindampfen des Filtrates wurde der Rückstand in Diäthyläther digeriert und lieferte 2,12 g 7-Amino-2,3,4,5-tetrahydro-1,3,3,5-tetramethyl-2,4-dioxo-1H-1,5-benzodiazepin, Smp. 221-222°C.

C. 1,58 g Terephthalsäuremonoäthylester wurden durch Rückflusserhitzen mit 1,5 ml Thionylchlorid in das entsprechende Säurechlorid überführt, das nach sorgfältigem Trocknen in 10 ml abs. Pyridin gelöst und zu einer Lösung von 2,01 g der in Absatz B. hergestellten Aminoverbindung in 15 ml abs. Pyridin bei 0°C getropft wurde. Nach 30 Minuten Reaktionszeit bei Raumtemperatur wurde auf Eis/konz. Salzsäure gegossen, mit Aethylacetat extrahiert, der Extrakt mit Wasser und gesättigter Natriumchloridlösung gewaschen, getrocknet und eingedampft. Umkristallisation aus Aethylacetat lieferte 2,89 g Aethyl-p-[(2,3,4,5-tetrahydro-1,3,3,5-tetramethyl-2,4-dioxo-1H-1,5-benzodiazepin-7-yl)carbamoyl]benzoat, Smp. >270°C.

## Beispiel 5

In Analogie zu Beispiel 1 wurde aus 7-Amino-2,3,4,5-tetrahydro-1,5,5-trimethyl-2-oxo-1H-1-benzazepin und p-Aethoxycarbonylbenzoylchlorid das Aethyl-p-[(2,3,4,5-tetrahydro-1,3,3-trimethyl-2-oxo-1-benzazepin-7-yl)carbamoyl]benzoat, Smp. 183-184 °C erhalten.

Beispiel 6

In Analogie zu Beispiel 1 wurde aus 7-Amino-2,3,4,5-tetrahydro-1-methyl-2-oxo-1H-1-benzazepin und p-Aethoxycarbonylbenzoylchlorid das Aethyl-p-[(2,3,4,5-tetrahydro-1-methyl-2-oxo-1H-1-benzazepin-7-yl)-carbamoyl]benzoat, Smp. 184,5-185 °C erhalten.

Beispiel 7

1,00 g Aethyl-p-[(2,3,4,5-tetrahydro-1,5,5-trimethyl-2-oxo-1H-1-benzazepin-7-yl)carbamoyl]benzoat in 75 ml Aethanol/Wasser (2:1) wurden mit 0,40 g Natriumhydroxid versetzt und das Reaktionsgemisch über Nacht bei Raumtemperatur gerührt. Danach wurde auf Eis/konz. Salzsäure gegossen, mit Aethylacetat extrahiert, der Extrakt mit gesättigter Natriumchloridlösung gewaschen, getrocknet und bis zur Kristallisation eingeengt. Man erhielt 0,79 g p-[(2,3,4,5-Tetrahydro-1,5,5-trimethyl-2-oxo-1H-1-benzazepin-7-yl)carbamoyl]-benzoesäure, Smp. >275 °C.

Beispiel 8

In Analogie zu Beispiel 7 wurden hergestellt:

p-[(2,3,4,5-Tetrahydro-1,3,3-trimethyl-2-oxo-1H-1-benzazepin-7-yl)carbamoyl]benzoesäure, Smp. 272-273 °C;

p-[(2,3,4,5-Tetrahydro-1-methyl-2-oxo-1H-1-benzazepin-7-yl)carbamoyl]benzoesäure, Smp. >270 °C;

rac-p-[(2,3,4,5-Tetrahydro-1,3,5,5-tetramethyl-2-oxo-1H-1-benzazepin-7-yl)carbamoyl]benzoesäure, Smp. >250 °C; und

p-[(2,3,4,5-Tetrahydro-1,3,3,5-tetramethyl-2,4-dioxo-1H-1,5-benzodiazepin-7-yl)carbamoyl]benzoesäure. Smp. >270 °C.

Beispiel 9

Zu 1,00 g Aethyl-p-(2,3,4,5-tetrahydro-1,5,5-trimethyl-2-oxo-1H-1-benzazepin-7-carboxamido)benzoat in 10 ml Aethanol/Tetrahydrofuran (1:1) wurden 1,69 ml 3N Natronlauge gegeben. Das Reaktionsgemisch wurde 3 Tage bei Raumtemperatur gerührt, danach auf Eis/konz. Salzsäure gegossen und mit Aethylacetat extrahiert. Der Extrakt wurde mit gesättigter Natriumchloridlösung gewaschen, getrocknet und eingedampft. Umkristallisation aus Aethylacetat lieferte 0,81 g p-(2,3,4,5-Tetrahydro-1,5,5-trimethyl-2-oxo-1H-1-benzazepin-7-carboxamido)benzoesäure, Smp. >275 °C.

Beispiel 10

In Analogie zu Beispiel 9 wurden hergestellt:

p-(2,3,4,5-Tetrahydro-1,3,3,5-tetramethyl-2,4-dioxo-1H-1,5-benzodiazepin-7-carboxamido)benzoesäure, Smp. >270 °C;

p-(2,3,4,5-Tetrahydro-1,3,3-trimethyl-2-oxo-1H-1-benzazepin-7-carboxamido)benzoesäure, Smp. 265-266 °C;

p-(2,3,4,5-Tetrahydro-1-methyl-2-oxo-1H-1-benzazepin-7-carboxamido)benzoesäure, Smp. >270 °C;

rac-p-(2,3,4,5-Tetrahydro-1,3-dimethyl-2-oxo-1H-1-benzazepin-7-carboxamido)benzoesäure, Smp. >280 °C;

rac-p-(2,3,4,5-Tetrahydro-1,3-dimethyl-2-oxo-1H-1-benzazepin-8-carboxamido)benzoesäure, Smp. >260 °C und

rac-p-(2,3,4,5-Tetrahydro-1,3,5,5-tetramethyl-2-oxo-1H-1-benzazepin-7-carboxamido)benzoesäure, Smp. >250 °C.

Beispiel 11

5 g 7-Brom-3,4-dihydro-4,4-dimethyl-1-propyl-2(1H)-chinolinon wurden in 40 ml Tributylamin gelöst und zusammen mit 15 g p-Aminobenzoesäureäthylester und 1 g Bis-triphenylphosphin-palladiumdichlorid in

einem Autoklaven unter einem Kohlenmonoxid-Druck von 15 bar während 64 Stunden auf 110°C erwärmt. Nach dem Abkühlen wurde vom Katalysator abfiltriert, mit Essigester verdünnt und mit eiskalter 3N Salzsäure und Wasser gewaschen und eingedampft. Durch Chromatographie (Kieselgel, Eluierungsmittel Hexan/Essigester 2:1) wurde der überschüssige p-Aminobenzoesäureäthylester abgetrennt. Nach Umkristallisation aus Essigester/Hexan erhielt man 5,2 g p-(1-Propyl-1,2,3,4-tetrahydro-4,4-dimethyl-2-oxo-7-chinolincarboxamido)benzoesäureäthylester, Smp. 142-144°C.

Das als Ausgangsprodukt verwendete 7-Brom-3,4-dihydro-4,4-dimethyl-1-propyl-2(1H)-chinolinon wurde wie folgt hergestellt:

44,3 g pulverisiertes Kaliumhydroxid wurden in 200 ml Dimethylsulfoxid suspendiert und eine Lösung von 50 g N-(3-Bromphenyl)-3,3-dimethyl-acrylsäureamid in 300 ml DMSO hinzugetropft. Man rührte 1 Stunde bei Raumtemperatur und tropfte anschliessend eine Lösung von 50,3 g Propyljodid in 200 ml DMSO hinzu. Nach 2 Stunden goss man das Reaktionsgemisch auf Eiswasser, extrahierte dreimal mit Essigester, wusch die organische Phase mehrfach mit Wasser, trocknete und dampfte ein. Nach Filtration des Rohproduktes über eine Kieselgelsäule (Eluierungsmittel Hexan/Essigester 9:1) erhielt man 58 g N-Propyl-N-(3-bromphenyl)-3,3-dimethyl-acrylamid als gelbes Oel. Dieses Produkt wurde in 1,5 l Petroläther (hochsiedend) gelöst. Unter Rühren gab man portionsweise 52,3 g Aluminiumchlorid dazu und erwärmte anschliessend während 2,5 Stunden am Rückfluss. Nach dem Abkühlen auf 0-5°C wurden 500 ml eiskalte 1N Salzsäure hinzugetropft, mit Wasser verdünnt und mehrfach mit Aether extrahiert. Das nach Trocknen und Eindampfen des Lösungsmittels erhaltene gelbe Oel war ein 6:4 Gemisch von 7-Brom- und 5-Brom-3,4-dihydro-4,4-dimethyl-1-propyl-2(1H)-chinolinon. Es wurde über eine Kieselgelsäule filtriert (Eluierungsmittel Hexan/5% Essigester) und in wenig Hexan aufgenommen. Nach Animpfen mit der 7-Brom-Verbindung kristallisierten in der Kälte 12,2 g reines 7-Brom-3,4-dihydro-4,4-dimethyl-1-propyl-2(1H)-chinolinon in farblosen Kristallen aus, Smp. 50-51°C.

## Beispiel 12

37,2 g 6-Amino-3,4-dihydro-1,4,4-trimethyl-2(1H)-chinolinon wurden in 600 ml Pyridin gelöst und bei Raumtemperatur tropfenweise mit einer Lösung von 43 g 4-Aethoxycarbonylbenzoylchlorid in 80 ml Tetrahydrofuran versetzt. Nach 20-stündigem Rühren bei Raumtemperatur wurde das Reaktionsgemisch auf Eiswasser gegossen, mit Essigester mehrfach extrahiert, die organische Phase mit kalter 2N Salzsäure und Wasser gewaschen, getrocknet und eingedampft. Nach Chromatographie des Rohproduktes (Kieselgel, Eluierungsmittel Hexan/Essigester 2:1, dann Essigester) und Umkristallisation aus Essigester/Hexan erhielt man 45,5 g p-[(1,2,3,4-Tetrahydro-1,4,4-trimethyl-2-oxo-6-chinolinyl)carbamoyl]benzoesäureäthylester, Smp. 197-199°C.

Die als Ausgangsmaterial verwendete Amino-Verbindung wurde wie folgt hergestellt:

50 g 3,4-Dihydro-1,4,4-trimethyl-2(1H)-chinolinon wurden in einem Gemisch aus 130 ml Essigsäureanhydrid und 42 ml Essigsäure gelöst. Bei einer Temperatur von 0-5°C tropfte man vorsichtig ein Gemisch von 105 ml Essigsäureanhydrid und 29 ml Salpetersäure 65% (hergestellt durch langsames Zutropfen der Salpetersäure zu Essigsäureanhydrid bei 0°C) hinzu. Nach 3-tägigem Rühren des Reaktionsgemisches bei Raumtemperatur goss man auf Eiswasser, extrahierte mit Essigester und wusch die organische Phase mit kalter, verdünnter Sodalösung und Wasser. Das nach Trocknen und Eindampfen der organischen Phase erhaltene, schwach bräunliche Oel kristallisierte beim Verreiben mit wenig Aether. Man erhielt 42 g 3,4-Dihydro-1,4,4-trimethyl-6-nitro-2(1H)-chinolinon, Smp. 125-127°C (aus Aethylacetat/Hexan). Dieses Produkt wurde in 2,5 l Aethanol gelöst und über Palladiumkohle (5%) unter Normaldruck bei Raumtemperatur hydriert. Nach Umkristallisation des Rohproduktes aus Essigester/Hexan erhielt man 34 g 6-Amino-3,4-dihydro-1,4,4-trimethyl-2(1H)-chinolinon, Smp. 126-128°C.

## Beispiel 13

40 g p-[(1,2,3,4-Tetrahydro-1,4,4-trimethyl-2-oxo-6-chinolinyl)carbamoyl]-benzoesäureäthylester wurden in 800 ml Aethanol suspendiert und mit einer Lösung von 60 g Kaliumhydroxid in 500 ml Wasser versetzt. Nach 3-stündigem Rühren bei 50°C erhielt man eine klare, gelbe Lösung. Das Reaktionsgemisch wurde zur Hälfte eingedampft, mit Eiswasser verdünnt und mit kalter 2N Salzsäure angesäuert. Die ausgefallene Carbonsäure wurde abgesaugt, mit Eiswasser gewaschen, am Hochvakuum bei 80°C getrocknet und aus 300 ml Essigsäure umkristallisiert. Man erhielt 31,5 g p-[(1,2,3,4-Tetrahydro-1,4,4-trimethyl-2-oxo-6-chinolinyl)carbamoyl]benzoesäure, Smp. 285-287°C.

## Beispiel 14

6 g 3,4-Dihydro-1,4,4-trimethyl-2-oxo-6-chinolincarbonsäure wurden mit 20 ml Thionylchlorid versetzt und während 2 Stunden am Rückfluss erwärmt. Nach dem Abdampfen des überschüssigen Thionylchlorides wurde der ölige Rückstand in 10 ml Tetrahydrofuran gelöst und langsam zu einer Lösung von 3,9 g 4-Amino-benzoesäureäthylester in 40 ml Pyridin hinzugetropft. Nach 20-stündigem Rühren bei Raumtemperatur wurde auf Eiswasser gegossen, mehrfach mit Essigester extrahiert, die organische Phase mit 2N Salzsäure gewaschen, getrocknet und eingedampft. Das ölige Rohprodukt wurde durch Chromatographie (Kieselgel, Eluierungsmittel Hexan/Essigester 2:1) gereinigt und aus Hexan/Essigester umkristallisiert. Man erhielt 4,9 g p-(1,2,3,4-Tetrahydro-1,4,4-trimethyl-2-oxo-6-chinolincarboxamido)benzoesäureäthylester, Smp. 175-177 ° C.

Die als Ausgangsprodukt verwendete 3,4-Dihydro-1,4,4-trimethyl-2-oxo-6-chinolincarbonsäure wurde wie folgt hergestellt:

19,7 g 6-Acetyl-3,4-dihydro-1,4,4-trimethyl-2(1H)-chinolinon wurden in 100 ml Dioxan gelöst und bei 0 ° C zu einer wässrigen Natriumhypobromidlösung (hergestellt durch Zutropfen von 17,2 ml Brom zu einer Lösung von 34,4 g Natriumhydroxid in 170 ml Wasser bei 5 ° C) hinzugetropft. Nach 1-stündigem Rühren bei Raumtemperatur bestand das Reaktionsgemisch aus zwei Phasen. Die obere Phase wurde verworfen, die untere nach Zugabe von 20 ml 10% Natriumbisulfitlösung durch Zugabe von konz. Salzsäure stark angesäuert. Mehrfache Extraktion mit Essigester ergab nach Trocknen und Eindampfen 17,9 g 3,4-Dihydro-1,4,4-trimethyl-2-oxo-6-chinolincarbonsäure, Smp. 195-197 ° C (aus Methylenchlorid/Hexan).

Beispiel 15

In Analogie zu Beispiel 13 erhielt man durch Verseifung von 2,6 g p-(1,2,3,4-Tetrahydro-1,4,4-trimethyl-2-oxo-6-chinolincarboxamido)benzoesäureäthylester mit wässriger Kaliumhydroxidlösung nach Umkristallisation aus Essigsäure 1,8 g p-(1,2,3,4-Tetrahydro-1,4,4-trimethyl-2-oxo-6-chinolincarboxamido)benzoesäure, Smp. >315 ° C (Zersetzung).

Beispiel 16

In Analogie zu Beispiel 11 wurden hergestellt:

Aethyl-p-(2,3,4,5-tetrahydro-1,5,5-trimethyl-2-oxo-1H-1-benzazepin-7-carboxamido)benzoat, Smp. 179-180 ° C;

Aethyl-p-(2,3,4,5-tetrahydro-1-methyl-2-oxo-1H-1-benzazepin-7-carboxamido)benzoat, Smp. 169-170 ° C;

Aethyl-rac-p-(2,3,4,5-tetrahydro-1,3-dimethyl-2-oxo-1H-1-benzazepin-8-carboxamido)benzoat, Smp. 223-224 ° C und

Aethyl-rac-p-(2,3,4,5-tetrahydro-1,3,5,5-tetramethyl-2-oxo-1H-1-benzazepin-7-carboxamido)benzoat, Smp. 185-187 ° C.

Beispiel 17

In Analogie zu Beispiel 13 erhielt man durch Verseifung von 4 g p-(1-Propyl-1,2,3,4-tetrahydro-4,4-dimethyl-2-oxo-7-chinolincarboxamido)benzoesäureäthylester nach Umkristallisation aus Essigester/Hexan 2,7g p-(1-Propyl-1,2,3,4-tetrahydro-4,4-dimethyl-2-oxo-7-chinolincarboxamido)benzoesäure, Smp. 215-217 ° C.

Beispiel 18

In Analogie zu Beispiel 11 wurde durch palladiumkatalysierte Carbonylierung von 7-Brom-1-decyl-3,4-dihydro-4,4-dimethyl-2(1H)-chinolinon und Reaktion mit p-Amino-benzoesäureäthylester nach Umkristallisation aus Diisopropyläther p-(1-Decyl-1,2,3,4-tetrahydro-4,4-dimethyl-2-oxo-chinolincarboxamido)-benzoesäureäthylester hergestellt, Smp. 95-96 ° C.

Die als Ausgangsprodukt verwendete Bromverbindung wurde in Analogie zu Beispiel 11 durch Alkylierung von N-(3-Bromphenyl)-3,3-dimethylacrylsäureamid mit Decylbromid und anschliessendem Ringschluss mit Aluminiumchlorid hergestellt. Die bei der Ringschlussreaktion anfallenden isomeren Bromverbindungen wurden in diesem Fall durch Mitteldruckchromatographie an Fertigsäulen Merck (Eluierungsmittel Hexan/Essigester 9:1) getrennt. Die 7-Brom-chinolinon-Verbindung hat den kleineren $R_F$-Wert und fällt als gelbes Oel an.

Beispiel 19

In Analogie zu Beispiel 13 erhielt man durch Verseifung von p-(1-Decyl-1,2,3,4-tetrahydro-4,4-dimethyl-2-oxo-chinolincarboxamido)benzoesäureäthylester nach Umkristallisation aus Essigester/Hexan p-(1-Decyl-1,2,3,4-tetrahydro-4,4-dimethyl-2-oxo-chinolincarboxamido)benzoesäure in weissen Kristallen, Smp. 161-163 ° C.

Beispiel 20

In Analogie zu Beispiel 11 wurde durch palladiumkatalysierte Carbonylierung von 7-Brom-3,4-dihydro-1,4,4-trimethy-2(1H)-chinolinon (Smp. 92-94 ° C) und Reaktion mit p-Amino-benzoesäureäthylester nach Umkristallisation aus Methylenchlorid/Aether p-(1,2,3,4-Tetrahydro-1,4,4-trimethyl-2-oxo-7-chinolincarboxamido)benzoesäureäthylester hergestellt, Smp. 129-130 ° C.

Beispiel 21

In Analogie zu Beispiel 13 erhielt man durch Verseifung von p-(1,2,3,4-Tetrahydro-1,4,4-trimethyl-2-oxo-7-chinolincarboxamido)benzoesäureäthylester nach Umkristallisation aus Essigester/Hexan p-(1,2,3,4-Tetrahydro-1,4,4-trimethyl-2-oxo-7-chinolincarboxamido)benzoesäure, Smp. 290-292 ° C.

Beispiel 22

A. 14,62 g 1-Tetralon in 45 ml Methylenchlorid wurden unter Eis/NaCl-Kühlung mit 29,3 ml Trifluoressigsäureanhydrid und 8,40 g Ammoniumnitrat versetzt. Nach 18 Stunden goss man vorsichtig auf Eis/konz. NaOH und extrahierte mit Diäthyläther. Die organische Phase wurde nacheinander mit Soda-Lösung und NaCl-Lösung gewaschen, getrocknet und eingedampft. Flash-Chromatographie an Kieselgel (Hexan/Aethylacetat = 9/2), gefolgt von Umkristallisation aus Hexan/Aethylacetat (1/1) lieferte 7,87 g 7-Nitro-1-tetralon als bräunliche Kristalle vom Smp. 104-105 ° C.

B. 7,87 g 7-Nitro-1-tetralon wurden in 55 ml AcOH vorgelegt. Man gab 7,0 g $NaN_3$ zu und erwärmte auf 60 ° C. Dann begann man, 10,3 ml $H_2SO_4$ konz. langsam zuzutropfen. $N_2$-Entwicklung setzte ein, die Temperatur stieg auf 90 ° C. Man hielt das Reaktionsgemisch 1/2 Stunde bei dieser Temperatur, kühlte dann ab und goss vorsichtig auf Eis/28% NaOH. Man extrahierte reichlich mit Aethylacetat, wusch mit wenig Wasser, trocknete und dampfte ein. Flash-Chromatographie an $SiO_2$ (Hexan/Aethylacetat (1/1) → Aethylacetat rein) lieferte 2,58 g 2,3,4,5-Tetrahydro-8-nitro-2-oxo-1H-1-benzazepin als braune Kristalle vom Smp. 224-226 ° C neben regioisomerem Lactam und nicht umgesetztem Ausgangsmaterial.

C. Zu 650 mg 55%iges NaH in 20 ml abs. Dimethylformamid gab man 2,58 g 2,3,4,5-Tetrahydro-8-nitro-2-oxo-1H-1-benzazepin, gelöst in 30 ml abs. Dimethylformamid. Man rührte 1 Stunde, kühlte dann auf 0 ° C und tropfte 1,35 ml Methyljodid langsam zu. Nach 45 Minuten goss man auf Eis, extrahierte mit Aethylacetat, wusch mit NaCl-Lösung, trocknete und dampfte ein. Flash-Chromatographie an $SiO_2$ -(Hexan/Aethylacetat = 1/1) lieferte 2,46 g 2,3,4,5-Tetrahydro-1-methyl-8-nitro-2-oxo-1H-1-benzazepin als orange Kristalle vom Smp. 151-152 ° C.

D. 1,34 g 2,3,4,5-Tetrahydro-1-methyl-8-nitro-2-oxo-1H-1-benzazepin wurden in 90 ml abs. Aethanol gelöst und über 150 mg Pd/C 5% bei Raumtemperatur und Atmosphärendruck über Nacht hydriert. Man filtrierte über eine Glasnutsche, dampfte ein und erhielt so 1,20 g 8-Amino-2,3,4,5-tetrahydro-1-methyl-2-oxo-1H-1-benzazepin als hellrosa Feststoff.

E. Aus 1,37 g Terephthalsäuremonoäthylester bereitete man durch Erhitzen zum Rückfluss mit 2,50 ml $SOCl_2$ das entsprechende Säurechlorid. Dieses wurde nach sorgfältiger Trocknung am Hochvakuum in 5 ml abs. Pyridin gelöst und bei 0 ° C zu 1,22 g 8-Amino-2,3,4,5-tetrahydro-1-methyl-2-oxo-1H-1-benzazepin getropft, das man in 5 ml abs. Pyridin vorgelegt hatte. Man liess 1/2 Stunde bei Raumtemperatur reagieren und goss dann auf Eis/HCl konz. Man extrahierte mit Aethylacetat und wusch nacheinander mit Soda-Lösung und NaCl-Lösung. Dann wurde der Extrakt getrocknet und im Vakuum auf ca. 15 ml Volumen eingeengt, wobei das Produkt auszufallen begann. Diese direkte Kristallisation lieferte 1,90 g Aethyl p-[(2,3,4,5-tetrahydro-1-methyl-2-oxo-1H-1-benzazepin-8-yl)carbamoyl]benzoat als weisse Kristalle vom Smp. 194,5-195,5 ° C.

Beispiel 23

850 g Aethyl p-[(2,3,4,5-tetrahydro-1-methyl-2-oxo-1H-1-benzazepin-8-yl)carbamoyl]benzoat wurden in 20 ml Tetrahydrofuran und 10 ml Aethanol gelöst und mit 10 ml 1,2N NaOH versetzt. Man rührte das zunächst 2-phasige Gemisch intensiv bei 35 ° C. Nach 8 Stunden goss man auf Eis/HCl konz. und

extrahierte reichlich mit Aethylacetat. Nach Waschen mit wenig NaCl-Lösung, Trocknen, Einengen, nochmaligem Aufkochen des Rückstandes in Aethylacetat und Trocknen wurden 400 mg p-[(2,3,4,5-Tetrahydro-1-methyl-2-oxo-1H-1-benzazepin-8-yl)carbamoyl]benzoesäure als beige Kristalle, Smp. >270 ° C erhalten.

Beispiel 24

In Analogie zu Beispiel 22 wurde, ausgehend von 4,4-Dimethyl-1-tetralon, Aethyl p-[(2,3,4,5-tetrahydro-1,5,5-trimethyl-2-oxo-1H-1-benzazepin-8-yl)carbamoyl]benzoat als farblose Kristalle vom Smp. 174-175 ° C erhalten.

Beispiel 25

In Analogie zu Beispiel 23 wurde, ausgehend von Aethyl p-[(2,3,4,5-tetrahydro-1,5,5-trimethyl-2-oxo-1H-1-benzazepin-8-yl)carbamoyl]benzoat, die p-[(2,3,4,5-Tetrahydro-1,5,5-trimethyl-2-oxo-1H-1-benzazepin-8-yl)carbamoyl]benzoesäure als farblose Kristalle vom Smp. >250 ° C erhalten.

Beispiel 26

In Analogie zu Beispiel 11 wurde, ausgehend von 7-Brom-3,4-dihydro-4,4-dimethyl-1-butyl-2(1H)-chinolinon, durch Pd-katalysierte Amidierung das Aethyl p-(-1-butyl-2,3,4,5-tetrahydro-5,5-dimethyl-2-oxo-1H-1-benzazepin-7-carboxamido)benzoat als beige Kristalle vom Smp. 137-138 ° C erhalten.

Beispiel 27

In Analogie zu Beispiel 9 wurde, ausgehend von Aethyl p-(1-butyl-2,3,4,5-tetrahydro-5,5-dimethyl-2-oxo-1H-1-benzazepin-7-carboxamido)benzoat die p-(1-Butyl-2,3,4,5-tetrahydro-5,5-dimethyl-2-oxo-1H-1-benzazepin-7-carboxamido)benzoesäure als farblose Kristalle vom Smp. 269-270 ° C erhalten.

Beispiel 28

In Analogie zu den vorstehenden Beispielen können die folgenden Verbindungen hergestellt werden:
Aethyl-p-[(1,2,3,4-tetrahydro-1,4,4-trimethyl-2-oxo-7-chinolinyl)carbamoyl]benzoat;
p-[(1,2,3,4-Tetrahydro-1,4,4-trimethyl-2-oxo-7-chinolinyl)carbamoyl]benzoesäure;
Aethyl-p-[(1,2,3,4-tetrahydro-1-propyl-4,4-dimethyl-2-oxo-7-chinolinyl)carbamoyl]benzoat;
p-[(1,2,3,4-Tetrahydro-1-propyl-4,4-dimethyl-2-oxo-7-chinolinyl)carbamoyl]benzoesäure;
Aethyl-p-(1,3,3-trimethyl-2-oxo-5-indolincarboxamido)benzoat;
Aethyl-p-[(1,3,3-trimethyl-2-oxo-5-indolinyl)carbamoyl]benzoat;
Aethyl-p-(1,3,3-trimethyl-2-oxo-6-indolincarboxamido)benzoat;
Aethyl-p-[(1,3,3-trimethyl-2-oxo-6-indolinyl)carbamoyl]benzoat;
Aethyl-p-[(2,3,4,5-tetrahydro-1,3,3-trimethyl-2-oxo-1H-1-benzazepin-8-yl)carbamoyl]benzoat;
Aethyl-p-(2,3,4,5-tetrahydro-1-methyl-2-oxo-1H-1-benzazepin-8-carboxamido)benzoat;
p-(2,3,4,5-Tetrahydro-1-methyl-2-oxo-1H-1-benzazepin-8-carboxamido)benzoesäure;
Aethyl-p-[(2,3,4,5-tetrahydro-1-n-butyl-5,5-dimethyl-2-oxo-1H-1-benzazepin-7-yl)carbamoyl]benzoat;
p-[(2,3,4,5-Tetrahydro-1-n-butyl-5,5-dimethyl-2-oxo-1H-1-benzazepin-7-yl)carbamoyl]benzoesäure.

Beispiel A

Hartgelatinekapseln können wie folgt hergestellt werden:

| Bestandteile | mg/Kapsel |
|---|---|
| 1. sprühgetrocknetes Pulver enthaltend 75% Verbindung I | 200 |
| 2. Natriumdioctylsulfosuccinat | 0,2 |
| 3. Natriumcarboxymethylcellulose | 4,8 |
| 4. mikrokristalline Cellulose | 86,0 |
| 5. Talk | 8,0 |
| 6. Magnesiumstearat | 1,0 |
| | Total 300 |

Das sprühgetrocknete Pulver, das auf dem Wirkstoff, Gelatine und mikrokristalliner Cellulose basiert und eine mittlere Korngrösse des Wirkstoffes von <1 μ aufweist (mittels Autokorrelationsspektroskopie gemessen), wird mit einer wässrigen Lösung von Natriumcarboxymethylcellulose und Natriumdioctylsulfosuccinat befeuchtet und geknetet. Die resultierende Masse wird granuliert, getrocknet und gesiebt, und das erhaltene Granulat mit mikrokristalliner Cellulose, Talk und Magnesiumstearat vermischt. Das Pulver wird in Kapseln der Grösse 0 abgefüllt.

Beispiel B

Tabletten können wie folgt hergestellt werden:

| Bestandteile | mg/Tablette |
|---|---|
| 1. Verbindung I als feingemahlenes Pulver | 500 |
| 2. Milchzucker pulv. | 100 |
| 3. Maisstärke weiss | 60 |
| 4. Povidone K30 | 8 |
| 5. Maisstärke weiss | 112 |
| 6. Talk | 16 |
| 7. Magnesiumstearat | 4 |
| | Total 800 |

Die feingemahlene Substanz wird mit Milchzucker und einem Teil der Maisstärke gemischt. Die Mischung wird mit einer wässrigen Lösung von Povidone K30 befeuchtet und geknetet, und die resultierende Masse granuliert, getrocknet und gesiebt. Das Granulat wird mit der restlichen Maisstärke, Talk und Magnesiumstearat vermischt und zu Tabletten geeigneter Grösse verpresst.

Beispiel C

Weichgelatinekapseln können wie folgt hergestellt werden:

| Bestandteile | mg/Kapsel |
|---|---|
| 1. Verbindung I | 50 |
| 2. Triglycerid | 450 |
| | Total 500 |

10 g Verbindung I werden unter Rühren, Inertbegasung und Lichtschutz in 90 g mittelkettigem Triglycerid gelöst. Diese Lösung wird als Kapselfüllmasse zu Weichgelatinekapseln à 50 mg Wirkstoff verarbeitet.

Beispiel D

Eine Lotion kann wie folgt hergestellt werden:

| Bestandteile | |
|---|---|
| 1. Verbindung I, feingemahlen | 3,0 g |
| 2. Carbopol 934 | 0,6 g |
| 3. Natriumhydroxid | q.s. ad pH 6 |
| 4. Aethanol, 94% | 50,0 g |
| 5. entmineralisiertes Wasser | ad 100,0 g |

Der Wirkstoff wird unter Lichtschutz in die Mischung Aethanol, 94%ig/Wasser eingearbeitet. Carbopol 934 wird bis zur vollständingen Gelierung eingerührt und der pH-Wert mit Natriumhydroxid eingestellt.

**Patentansprüche**

**1.** Verbindungen der Formel

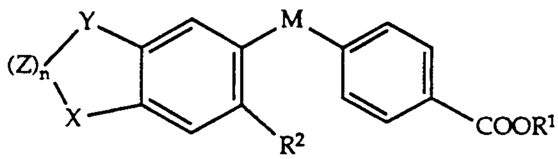

I

worin

| | |
|---|---|
| $R^1$ | Wasserstoff, nieder-Alkyl oder ein Kation; |
| $R^2$ | Wasserstoff, nieder-Alkyl, nieder-Alkoxy oder Halogen; |
| M | -CONH- oder -NHCO-; |
| X und Y | $>CR^3R^4$ oder $-CONR^5-$; |
| Z | $>CR^6R^7$; |
| $R^3$, $R^4$, $R^6$ und $R^7$ | Wasserstoff oder nieder-Alkyl; |
| $R^5$ | Alkyl; und |
| n | 0,1 oder 2 bedeuten; |

wobei mindestens ein Rest X oder Y $-CONR^5-$ ist und dieser Rest über das N-Atom an den Phenylring gebunden ist,
und pharmazeutisch anwendbaren Salzen von Carbonsäuren der Formel I.

**2.** Verbindungen gemäss Anspruch 1, in denen n 1 oder 2 ist.

**3.** Verbindungen gemäss Anspruch 1 oder 2, in denen X $-CONR^5-$ und Y $>CR^6R^7$ ist.

**4.** Die Verbindung gemäss Anspruch 3, p-[(1,2,3,4-Tetrahydro-1,4,4-trimethyl-2-oxo-6-chinolinyl)-carbamoyl]benzoesäure.

**5.** Die Verbindungen gemäss Anspruch 3,

Aethyl-p-[(2,3,4,5-tetrahydro-1,5,5-trimethyl-2-oxo-1H-1-benzazepin-7-yl)carbamoyl]benzoat,
Aethyl-p-(2,3,4,5-tetrahydro-1,3,3-trimethyl-2-oxo-1H-1-benzazepin-7-carboxamido)benzoat,
Aethyl-p-[(2,3,4,5-tetrahydro-1,3,3-trimethyl-2-oxo-1-benzazepin-7-yl)carbamoyl]benzoat,
Aethyl-p-[(2,3,4,5-tetrahydro-1-methyl-2-oxo-1H-1-benzazepin-7-yl)carbamoyl]benzoat,
p-[(2,3,4,5-Tetrahydro-1,5,5-trimethyl-2-oxo-1H-1-benzazepin-7-yl)carbamoyl]benzoesäure,
p-[(2,3,4,5-Tetrahydro-1,3,3-trimethyl-2-oxo-1H-1-benzazepin-7-yl)carbamoyl]benzoesäure,
p-[(2,3,4,5-Tetrahydro-1-methyl-2-oxo-1H-1-benzazepin-7-yl)carbamoyl]benzoesäure,
rac-p-[(2,3,4,5-Tetrahydro-1,3,5,5-tetramethyl-2-oxo-1H-1-benzazepin-7-yl)carbamoyl]benzoesäure,
p-(2,3,4,5-Tetrahydro-1,5,5-trimethyl-2-oxo-1H-1-benzazepin-7-carboxamido)benzoesäure,
p-(2,3,4,5-Tetrahydro-1,3,3-trimethyl-2-oxo-1H-1-benzazepin-7-carboxamido)benzoesäure,
p-(2,3,4,5-Tetrahydro-1-methyl-2-oxo-1H-1-benzazepin-7-carboxamido)benzoesäure,
rac-p-(2,3,4,5-Tetrahydro-1,3-dimethyl-2-oxo-1H-1-benzazepin-7-carboxamido)benzoesäure,
rac-p-(2,3,4,5-Tetrahydro-1,3-dimethyl-2-oxo-1H-1-benzazepin-8-carboxamido)benzoesäure,

p-[(1,2,3,4-Tetrahydro-1,4,4-trimethyl-2-oxo-6-chinolinyl)carbamoyl]benzoesäureäthylester,
p-(1,2,3,4-Tetrahydro-1,4,4-trimethyl-2-oxo-6-chinolincarboxamido)benzoesäureäthylester,
p-(1,2,3,4-Tetrahydro-1,4,4-trimethyl-2-oxo-6-chinolincarboxamido)benzoesäure,
Aethyl-p-(2,3,4,5-tetrahydro-1,5,5-trimethyl-2-oxo-1H-1-benzazepin-7-carboxamido)benzoat,
Aethyl-p-(2,3,4,5-tetrahydro-1-methyl-2-oxo-1H-1-benzazepin-7-carboxamido)benzoat,
Aethyl-rac-p-(2,3,4,5-tetrahydro-1,3,5,5-tetramethyl-2-oxo-1H-1-benzazepin-7-carboxamido)benzoat,
Aethyl p-(1-butyl-2,3,4,5-tetrahydro-5,5-dimethyl-2-oxo-1H-1-benzazepin-7-carboxamido)benzoat,
p-(1-Butyl-2,3,4,5-tetrahydro-5,5-dimethyl-2-oxo-1H-1-benzazepin-7-carboxamido)benzoesäure.

**6.** Verbindungen gemäss Anspruch 1, in denen Y $-CONR^5-$ und X $>CR^6R^7$ ist.

**7.** Die Verbindungen gemäss Anspruch 6,

rac-p-(2,3,4,5-Tetrahydro-1,3,5,5-tetramethyl-2-oxo-1H-1-benzazepin-7-carboxamido)benzoesäure,
p-(1-Propyl-1,2,3,4-tetrahydro-4,4-dimethyl-2-oxo-7-chinolincarboxamido)benzoesäureäthylester,
Aethyl-rac-p-(2,3,4,5-tetrahydro-1,3-dimethyl-2-oxo-1H-1-benzazepin-8-carboxamido)benzoat,
p-(1-Propyl-1,2,3,4-tetrahydro-4,4-dimethyl-2-oxo-7-chinolincarboxamido)benzoesäure,
p-(1-Decyl-1,2,3,4-tetrahydro-4,4-dimethyl-2-oxo-chinolincarboxamido)benzoesäureäthylester,
p-(1-Decyl-1,2,3,4-tetrahydro-4,4-dimethyl-2-oxo-chinolincarboxamido)benzoesäure,
p-(1,2,3,4-Tetrahydro-1,4,4-trimethyl-2-oxo-7-chinolincarboxamido)benzoesäureäthylester,
p-(1,2,3,4-Tetrahydro-1,4,4-trimethyl-2-oxo-7-chinolincarboxamido)benzoesäure,
Aethyl p-[(2,3,4,5-tetrahydro-1-methyl-2-oxo-1H-1-benzazepin-8-yl)carbamoyl]benzoat,
p-[(2,3,4,5-Tetrahydro-1-methyl-2-oxo-1H-1-benzazepin-8-yl)carbamoyl]benzoesäure,
Aethyl p-[(2,3,4,5-tetrahydro-1,5,5-trimethyl-2-oxo-1H-1-benzazepin-8-yl)carbamoyl]benzoat,
p-[(2,3,4,5-Tetrahydro-1,5,5-trimethyl-2-oxo-1H-1-benzazepin-8-yl)carbamoyl]benzoesäure.

**8.** Die Verbindungen gemäss Anspruch 1 oder 2, in denen X und Y $-CONR^5-$ sind.

**9.** Die Verbindungen gemäss Anspruch 8,

Aethyl-p-(2,3,4,5-tetrahydro-1,3,3,5-tetramethyl-2,4-dioxo-1H-1,5-benzodiazepin-7-carboxamido)-benzoat,
Aethyl-p-[(2,3,4,5-tetrahydro-1,3,3,5-tetramethyl-2,4-dioxo-1H-1,5-benzodiazepin-7-yl)carbamoyl]-benzoat,
p-[(2,3,4,5-Tetrahydro-1,3,3,5-tetramethyl-2,4-dioxo-1H-1,5-benzodiazepin-7-yl)carbamoyl]-benzoesäure,
p-(2,3,4,5-Tetrahydro-1,3,3,5-tetramethyl-2,4-dioxo-1H-1,5-benzodiazepin-7-carboxamido)-benzoesäure.

**10.** Die Verbindungen der Ansprüche 1-9 zur Anwendung als Heilmittel.

**11.** Pharmazeutische Präparate, enthaltend eine Verbindung der Formel I und übliche pharmazeutische Hilfsstoffe.

**12.** Verwendung von Verbindungen der Formel I zur Herstellung pharmazeutischer Präparate für die Therapien und Prophylaxe von entzündlichen, allergischen, rheumatischen und immunologischen Erkrankungen, insbesondere Akne und Psoriasis; sowie Altershaut.

**13.** Verfahren zur Herstellung der Verbindungen von Anspruch 1, dadurch gekennzeichnet, dass man eine Verbindung der Formel

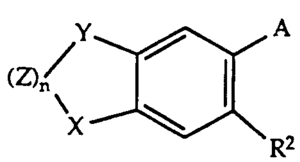

II

mit einer Verbindung der Formel

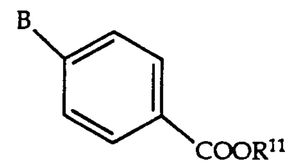

III

umsetzt;
oder eine Verbindung der Formel

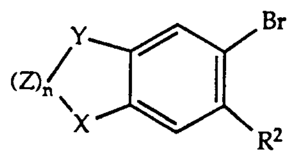

IV

mit einer Verbindung der Formel

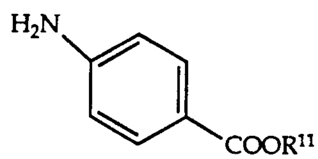

V

und Kohlenmonoxid in Gegenwart eines Uebergangsmetalls und eines Amins umsetzt,

wobei entweder A eine Carboxylgruppe oder ein reaktionsfähiges Derivat davon und B eine Aminogruppe bedeutet, oder A eine Aminogruppe und B eine Carboxylgruppe oder ein reaktionsfähiges Derivat davon und $R^{11}$ nieder-Alkyl bedeutet,
und gewünschtenfalls die Estergruppe $COOR^{11}$ verseift und die gebildete Säure als solche oder als Salz isoliert.

**Patentansprüche für folgende Vertragsstaaten : GR, ES**

**1.** Verfahren zur Herstellung von Verbindungen der Formel

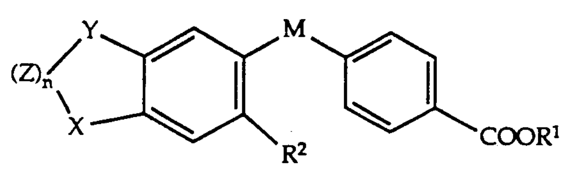

I

worin

| | |
|---|---|
| $R^1$ | Wasserstoff, nieder-Alkyl oder ein Kation; |
| $R^2$ | Wasserstoff, nieder-Alkyl, nieder-Alkoxy oder Halogen; |
| M | -CONH- oder -NHCO; |
| X und Y | $>CR^3R^4$ oder $-CONR^5-$; |
| Z | $>CR^6R^7$; |
| $R^3$, $R^4$, $R^6$ und $R^7$ | Wasserstoff oder nieder-Alkyl; |
| $R^5$ | Alkyl; und |
| n | 0,1 oder 2 bedeuten; |

wobei mindestens ein Rest X oder Y $-CONR^5-$ ist und dieser Rest über das N-Atom an den Phenylring gebunden ist, und pharmazeutisch anwendbaren Salzen von Carbonsäuren der Formel I,

dadurch gekennzeichnet, dass man eine Verbindung der Formel

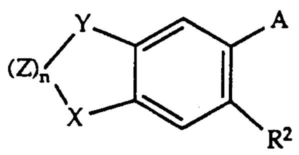

II

mit einer Verbindung der Formel

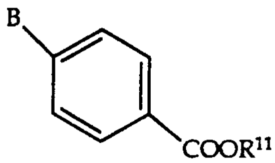

III

umsetzt;
oder eine Verbindung der Formel

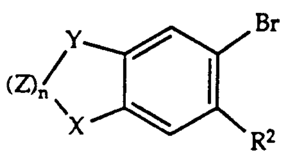

IV

mit einer Verbindung der Formel

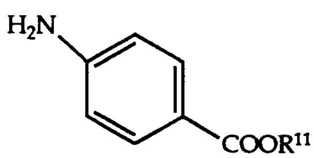

V

und Kohlenmonoxid in Gegenwart eines Uebergangsmetalls und eines Amins umsetzt,

wobei entweder A eine Carboxylgruppe oder ein reaktionsfähiges Derivat davon und B eine Aminogruppe bedeutet, oder A eine Aminogruppe und B eine Carboxylgruppe oder ein reaktionsfähiges Derivat davon und $R^{11}$ nieder-Alkyl bedeutet,
und gewünschtenfalls die Estergruppe $COOR^{11}$ verseift und die gebildete Säure als solche oder als Salz isoliert.

2. Verfahren gemäss Anspruch 1, wobei n 1 oder 2 ist.

3. Verfahren gemäss Anspruch 1 oder 2, wobei X $-CONR^5-$ und Y $>CR^6R^7$ ist.

4. Verfahren gemäss Anspruch 3 zur Herstellung von p-[(1,2,3,4-Tetrahydro-1,4,4-trimethyl-2-oxo-6-chinolinyl)carbamoyl]benzoesäure.

5. Verfahren gemäss Anspruch 3 zur Herstellung von

Aethyl-p-[(2,3,4,5-tetrahydro-1,5,5-trimethyl-2-oxo-1H-1-benzazepin-7-yl)carbamoyl]benzoat,
Aethyl-p-(2,3,4,5-tetrahydro-1,3,3-trimethyl-2-oxo-1H-1-benzazepin-7-carboxamido)benzoat,
Aethyl-p-[(2,3,4,5-tetrahydro-1,3,3-trimethyl-2-oxo-1-benzazepin-7-yl)carbamoyl]benzoat,
Aethyl-p-[(2,3,4,5-tetrahydro-1-methyl-2-oxo-1H-1-benzazepin-7-yl)carbamoyl]benzoat,

p-[(2,3,4,5-Tetrahydro-1,5,5-trimethyl-2-oxo-1H-1-benzazepin-7-yl)carbamoyl]benzoesäure,
p-[(2,3,4,5-Tetrahydro-1,3,3-trimethyl-2-oxo-1H-1-benzazepin-7-yl)carbamoyl]benzoesäure,
p-[(2,3,4,5-Tetrahydro-1-methyl-2-oxo-1H-1-benzazepin-7-yl)carbamoyl]benzoesäure,
rac-p-[(2,3,4,5-Tetrahydro-1,3,5,5-tetramethyl-2-oxo-1H-1-benzazepin-7-yl)carbamoyl]benzoesäure,
p-(2,3,4,5-Tetrahydro-1,5,5-trimethyl-2-oxo-1H-1-benzazepin-7-carboxamido)benzoesäure,
p-(2,3,4,5-Tetrahydro-1,3,3-trimethyl-2-oxo-1H-1benzazepin-7-carboxamido)benzoesäure,
p-(2,3,4,5-Tetrahydro-1-methyl-2-oxo-1H-1-benzazepin-7-carboxamido)benzoesäure,
rac-p-(2,3,4,5-Tetrahydro-1,3-dimethyl-2-oxo-1H-1-benzazepin-7-carboxamido)benzoesäure,
rac-p-(2,3,4,5-Tetrahydro-1,3-dimethyl-2-oxo-1H-1-benzazepin-8-carboxamido)benzoesäure,
p-[(1,2,3,4-Tetrahydro-1,4,4-trimethyl-2-oxo-6-chinolinyl)carbamoyl]benzoesäureäthylester,
p-(1,2,3,4-Tetrahydro-1,4,4-trimethyl-2-oxo-6-chinolincarboxamido)benzoesäureäthylester,
p-(1,2,3,4-Tetrahydro-1,4,4-trimethyl-2-oxo-6-chinolincarboxamido)benzoesäure,
Aethyl-p-(2,3,4,5-tetrahydro-1,5,5-trimethyl-2-oxo-1H-1-benzazepin-7-carboxamido)benzoat,
Aethyl-p-(2,3,4,5-tetrahydro-1-methyl-2-oxo-1H-1-benzazepin-7-carboxamido)benzoat,
Aethyl-rac-p-(2,3,4,5-tetrahydro-1,3,5,5-tetramethyl-2-oxo-1H-1-benzazepin-7-carboxamido)benzoat.

**6.** Verfahren gemäss Anspruch 1, wobei Y $-CONR^5-$ und X $>CR^6R^7$ ist.

**7.** Verfahren gemäss Anspruch 6 zur Herstellung von

rac-p-(2,3,4,5-Tetrahydro-1,3,5,5-tetramethyl-2-oxo-1H-1-benzazepin-7-carboxamido)benzoesäure,
p-(1-Propyl-1,2,3,4-tetrahydro-4,4-dimethyl-2-oxo-7-chinolincarboxamido)benzoesäureäthylester,
Aethyl-rac-p-(2,3,4,5-tetrahydro-1,3-dimethyl-2-oxo-1H-1-benzazepin-8-carboxamido)benzoat,
p-(1-Propyl-1,2,3,4-tetrahydro-4,4-dimethyl-2-oxo-7-chinolincarboxamido)benzoesäure,
p-(1-Decyl-1,2,3,4-tetrahydro-4,4-dimethyl-2-oxo-chinolincarboxamido)benzoesäureäthylester,
p-(1-Decyl-1,2,3,4-tetrahydro-4,4-dimethyl-2-oxo-chinolincarboxamido)benzoesäure,
p-(1,2,3,4-Tetrahydro-1,4,4-trimethyl-2-oxo-7-chinolincarboxamido)benzoesäureäthylester,
p-(1,2,3,4-Tetrahydro-1,4,4-trimethyl-2-oxo-7-chinolincarboxamido)benzoesäure.

**8.** Verfahren gemäss Anspruch 1 oder 2, wobei X und Y $-CONR^5-$ sind.

**9.** Verfahren gemäss Anspruch 8 zur Herstellung von

Aethyl-p-(2,3,4,5-tetrahydro-1,3,3,5-tetramethyl-2,4-dioxo-1H-1,5-benzodiazepin-7-carboxamido)-benzoat,
Aethyl-p-[(2,3,4,5-tetrahydro-1,3,3,5-tetramethyl-2,4-dioxo-1H-1,5-benzodiazepin-7-yl)carbamoyl]-benzoat,
p-[(2,3,4,5-Tetrahydro-1,3,3,5-tetramethyl-2,4-dioxo-1H-1,5-benzodiazepin-7-yl)carbamoyl]-benzoesäure,
p-(2,3,4,5-Tetrahydro-1,3,3,5-tetramethyl-2,4-dioxo-1H-1,5-benzodiazepin-7-carboxamido)-benzoesäure.

**10.** Verfahren zur Herstellung pharmazeutischer Präparate, dadurch gekennzeichnet, dass man eine Verbindung der Formel I oder von pharmazeutisch anwendbaren Salzen von Carbonsäuren der Formel I mit üblichen pharmazeutischen Hilfs- und Trägerstoffen vermischt und in eine zur therapeutischen Anwendung geeignete Form bringt.

**11.** Verwendung von Verbindungen der Formel I oder von pharmazeutisch anwendbaren Salzen von Carbonsäuren der Formel I zur Herstellung pharmazeutischer Präparate für die Therapien und Prophylaxe von entzündlichen, allergischen, rheumatischen und immunologischen Erkrankungen, insbesondere Akne und Psoriasis; sowie Altershaut.

Europäisches Patentamt

# EUROPÄISCHER RECHERCHENBERICHT

Nummer der Anmeldung

EP 92 10 3970

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 327 986 (BOEHRINGER MANNHEIM GMBH)<br>* das ganze Dokument, insbesondere Seite 5, Zeilen 26-31 *<br>--- | 1-13 | C07D209/34<br>C07D215/38<br>C07D215/48<br>C07D223/16<br>C07D241/44<br>C07D243/12<br>C07D245/06<br>A61K31/40<br>A61K31/47<br>A61K31/495<br>A61K31/55 |
| A | EP-A-0 350 846 (HOFFMANN-LA ROCHE AG)<br>* das ganze Dokument, insbesondere Seite 24, Beispiele 60 und 61, Seite 27, Zeilen 11, 12 und 20 und Seite 31, Zeilen 29-32 *<br>----- | 1-13 | |

RECHERCHIERTE SACHGEBIETE (Int. Cl.5)

C07D

Der vorliegende Recherchenbericht wurde für alle Patentansprüche erstellt

| Recherchenort | Abschlußdatum der Recherche | Prüfer |
|---|---|---|
| DEN HAAG | 15 JUNI 1992 | ALLARD M.S. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument